# FASCICULE DE BREVET EUROPEEN

(11) **EP 1 040 817 B1**
(45) Date de publication et mention de la délivrance du brevet: **02.02.2005**
(21) Numéro de dépôt: 00400544.3
(22) Date de dépôt: 29.02.2000
(51) Int. Cl.: A61K 7/13

(54) **Composition tinctoriale contenant une pyrazolo-(1,5-a)-pyrimidine et une polyaminopyrimidine monocyclique à titre de bases d'oxydation et un coupler, et procédé de teinture**
Färbemittel enthaltend Pyrazolo(1,5-a)pyrimidine und monocyclische Polyaminopyrimidine als Oxidationsmittel und einen Kuppler, sowie Färbeverfahren
Dyeing composition comprising a pyrazolo(1,5-a)pyrimidine and a monocyclic polyaminopyrimidine as oxidation bases and a coupler, and dyeing process

(30) Priorité: 29.03.1999 FR 9903889
(43) Date de publication de la demande: 04.10.2000
(73) Titulaire: L'OREAL, 75008 Paris (FR)
(72) Inventeur: Audousset, Marie-Pascale, 92600 Asnieres (FR)
(74) Mandataire: Goulard, Sophie

(56) Documents cités:
- WO-A-98/55083
- DE-A- 4 029 324
- FR-A- 2 746 309
- FR-A- 2 750 048
- FR-A- 2 771 631
- FR-A- 2 779 949
- US-A- 5 176 716

## Description

L'invention a pour objet une composition pour la teinture d'oxydation des fibres kératiniques contenant au moins une pyrazolo-[1,5-a]-pyrimidine à titre de première base d'oxydation, au moins une polyaminopyrimidine monocyclique à titre de seconde base d'oxydation, et au moins un coupleur ; ainsi que le procédé de teinture d'oxydation mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bis-phénylalkylènediamines ou bien encore des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, en particulier dans la demande de brevet FR-A-2 750 048, d'utiliser des pyrazolo-[1,5-a]-pyrimidines à titre de base d'oxydation, seules ou en association avec un ou plusieurs coupleurs. Cependant, les colorations obtenues ne sont pas toujours assez puissantes, chromatiques ou résistantes aux différentes agressions que peuvent subir les cheveux.

Or, la Demanderesse vient maintenant de découvrir, de façon totalement inattendue et surprenante, que l'association des pyrazolo-[1,5-a]-pyrimidines de formule (I) ci-après définie à titre de première base d'oxydation, d'au moins une polyaminopyrimidine monocyclique à titre de seconde base d'oxydation et d'au moins un coupleur permettait d'obtenir des colorations puissantes, chromatiques, et présentant de plus des propriétés de résistance améliorées vis à vis des diverses agressions que peuvent subir les cheveux (shampooings, lumière, intempéries, ondulations permanentes, transpiration, frottements, etc...).

Ces découvertes sont à la base de la présente invention.

L'invention a donc pour premier objet une composition pour la teinture d'oxydation des fibres kératiniques, et en particulier des fibres kératiniques humaines telles que les cheveux, caractérisée par le fait qu'elle contient, dans un milieu approprié pour la teinture :
- au moins une base d'oxydation choisie parmi les pyrazolo-[1,5-a]-pyrimidines de formule (I) suivante et leurs sels d'addition avec un acide ou avec une base : 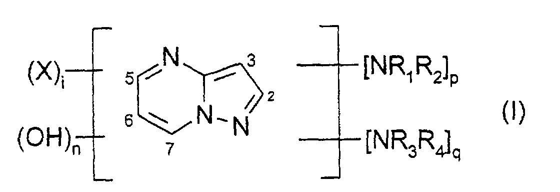 dans laquelle :
- R₁, R₂ R₃ et R₄ désignent , identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'amine pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl- ou di-[hydroxy(C₁-C₄) alkyl]-amino alkyle en C₁-C₄ ;
- les radicaux X, identiques ou différents, désignent un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
   sous réserve que :
- (i) la somme p + q est différente de 0 ;
- (ii) lorsque p + q est égal à 2, alors n vaut 0 et les groupes NR₁R₂ et NR₃R₄ occupent les positions (2,3) ; (5,6) ; (6,7) ; (3,5) ou (3,7) ;
- (iii) lorsque p + q est égal à 1 alors n vaut 1 et le groupe NR₁R₂ (ou NR₃R₄) et le groupe OH occupent les positions (2,3) ; (5,6) ; (6,7) ; (3,5) ou (3,7) ;
- au moins une polyaminopyrimidine monocyclique à titre de seconde base d'oxydation ;
- et au moins un coupleur.

Comme indiqué précédemment, la composition tinctoriale conforme à l'invention conduit à des colorations puissantes et chromatiques qui présentent de plus d'excellentes propriétés de résistance vis à vis de l'action des différents agents extérieurs (lumière, intempéries, lavage, ondulation permanente, transpiration, frottements).

L'invention a également pour objet un procédé de teinture d'oxydation des fibres kératiniques mettant en oeuvre cette composition tinctoriale.

Les pyrazolo-[1,5-a]-pyrimidines de formule (I) utilisables à titre de base d'oxydation dans la composition tinctoriale conforme à l'invention sont des composés connus et décrits dans la demande de brevet FR-A-2 750 048.

Parmi les pyrazolo-[1,5-a]-pyrimidines de formule (I), utilisables à titre de base d'oxydation dans les compositions tinctoriales conformes à l'invention, on peut notamment citer :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
- la 3-amino-5-méthyl-7-imidazolylpropylamino pyrazolo-[1,5-a]-pyrimidine ; et leurs sels d'addition avec un acide ou avec une base.

Parmi les polyaminopyrimidines monocycliques utilisables à titre de seconde base d'oxydation dans la composition tinctoriale conforme à l'invention, on peut notamment citer les composés décrits par exemple dans les brevets allemand DE 2 359 399 ou japonais JP 88-169 571 et JP 91-333 495 ou demandes de brevet WO 96/15765, et EP 0 467 026 comme la 2,4,5,6-tétra-aminopyrimidine, la 4-hydroxy 2,5,6-triaminopyrimidine, la 6-hydroxy 2,4,5-triaminopyrimidine, la 2-hydroxy 4,5,6-triaminopyrimidine, la 2,4-dihydroxy 5,6-diaminopyrimidine, la 2,6-dihydroxy 4,5-diaminopyrimidine, la 4,6-dihydroxy 2,5-diaminopyrimidine, la 2,5,6-triaminopyrimidine, la 2-diméthylamino 4,5,6-triaminopyrimidine, et leurs sels d'addition avec un acide.

La nature du ou des coupleurs pouvant être utilisés dans la composition tinctoriale conforme à l'invention n'est pas critique. Ils peuvent être choisis parmi les coupleurs classiquement utilisés pour la teinture des fibres kératiniques et parmi lesquels on peut notamment citer les métaphénylènediamines, les métaaminophénols, les métadiphénols et les coupleurs hétérocycliques tels que par exemple les dérivés indoliques, les dérivés indoliniques, les dérivés pyridiniques, les pyrazolones, et leurs sels d'addition avec un acide.

Ces coupleurs sont plus particulièrement choisis parmi le 5-amino 2-méthyl phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 6-hydroxybenzomorpholine, la 2,6-dihydroxy 4-méthyl pyridine, le 1-H 3-méthyl pyrazole 5-one, le 1-phényl 3-méthyl pyrazole 5-one, et leurs sels d'addition avec un acide.

D'une manière générale, les sels d'addition avec un acide utilisables dans le cadre de l'invention (bases d'oxydation et coupleurs) sont notamment choisis parmi les chlorhydrates, les bromhydrates, les sulfates, les citrates, les succinates, les tartrates, les lactates et les acétates. Les sels d'addition avec une base utilisables dans le cadre des compositions tinctoriales de l'invention sont notamment ceux obtenus avec la soude, la potasse, l'ammoniaque ou les amines.

Selon l'invention, la ou les pyrazolo-[1,5-a]-pyrimidines de formule (I) et/ou le ou leurs sels d'addition avec un acide ou avec une base représentent de préférence de 0,0005 à 12 % en poids du poids total de la composition tinctoriale, et encore plus préférentiellement de 0,005 à 6 % en poids de ce poids.

Selon l'invention, la ou les polyaminopyrimidines monocycliques et/ou le ou leurs sels d'addition avec un acide représentent de préférence de 0,0005 à 12 % en poids du poids total de la composition tinctoriale et encore plus préférentiellement de 0,005 à 6 % en poids de ce poids.

Le ou les coupleurs représentent de préférence de 0,0001% à 10% en poids du poids total de la composition tinctoriale et encore plus préférentiellement de 0,005 à 5 % en poids de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (II) suivante : 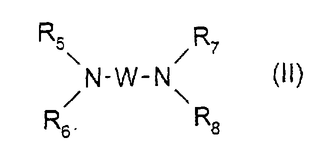 dans laquelle W est un reste propylène éventuellement substitué par un groupement hydroxyle ou un radical alkyle en C₁-C₆ ; R₅, R₆, R₇ et R₈, identiques ou différents, représentent un atome d'hydrogène, un radical alkyle en C₁-C₆ ou hydroxyalkyle en C₁-C₆.

La composition tinctoriale conforme à l'invention peut également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques tels que par exemple des gommes de guar non-ioniques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de poudres, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides, les enzymes telles que les oxydo-réductases à 2 électrons, les peroxydases et les laccases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples suivants sont destinés à illustrer l'invention.

### EXEMPLES

### EXEMPLES 1 et 3 DE TEINTURE EN MILIEU ALCALIN

On a préparé les compositions tinctoriales conformes à l'invention suivantes, (teneurs en grammes) :

| **EXEMPLE** | **1** | **2** |
|---|---|---|
| 3,7-diaminopyrazolopyrimidine, 2HCl (base d'oxydation de formule (I)) | 0,666 | 0,666 |
| 2,4,5,6-tetraaminopyrimidine, sulfate | 0,714 | - |
| 2,5,6-triamino 4-hydroxypyrimidine, sulfate | - | 0,777 |
| 1,3-dihydroxy benzène (coupleur) | 0,66 | - |
| 5-amino 2-méthyl phénol (coupleur) | - | 0,738 |
| Support de teinture commun | (*) | (*) |
| Eau déminéralisée q.s.p. | 100 g | 100 g |

| | | |
|---|---|---|
| (*) Support de teinture commun : | | |

- Alcool éthylique à 96° 18 g
- Métabisulfite de sodium en solution aqueuse à 35% 0,68 g
- Sel pentasodique de l'acide diéthylènetriaminopentacétique 1,1 g
- Ammoniaque à 20% de NH₃ 10,0 g

Au moment de l'emploi, on a mélangé poids pour poids chacune des compositions tinctoriales ci-dessus avec une solution de peroxyde d'hydrogène à 20 volumes (6% en poids) de pH 3.

Le mélange obtenu a été appliqué sur des mèches de cheveux gris naturels à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **1** | 10 | Marron cuivré |
| **2** | 10 | Irisé rouge |

## Revendications

1. Composition pour la teinture d'oxydation des fibres kératiniques, et en particulier des fibres kératiniques humaines telles que les cheveux, **caractérisée par le fait qu'**elle contient, dans un milieu approprié pour la teinture :
- au moins une base d'oxydation choisie parmi les pyrazolo-[1,5-a]-pyrimidines de formule (I) suivante et leurs sels d'addition avec un acide ou avec une base : 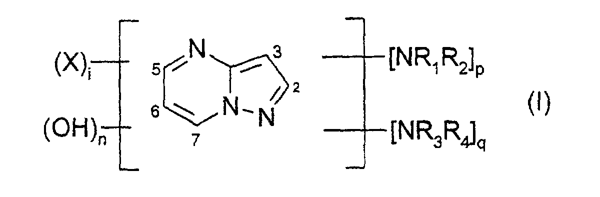 dans laquelle :
- R₁, R₂ R₃ et R₄ désignent , identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'amine pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl- ou di-[hydroxy(C₁-C₄)alkyl]-amino alkyle en C₁-C₄ ;
- les radicaux X, identiques ou différents, désignent un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1;
sous réserve que :
- (i) la somme p + q est différente de 0 ;
- (ii) lorsque p + q est égal à 2, alors n vaut 0 et les groupes NR₁R₂ et NR₃R₄ occupent les positions (2,3) ; (5,6) ; (6,7) ; (3,5) ou (3,7) ;
- (iii) lorsque p + q est égal à 1 alors n vaut 1 et le groupe NR₁R₂ (ou NR₃R₄) et le groupe OH occupent les positions (2,3) ; (5,6) ; (6,7) ; (3,5) ou (3,7) ;
- au moins une polyaminopyrimidine monocyclique à titre de seconde base d'oxydation ;
- et au moins un coupleur.

2. Composition selon la revendication 1, **caractérisée par le fait que** les pyrazolo-[1,5-a]-pyrimidines de formule (I) sont choisies parmi :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
- la 3-amino-5-méthyl-7-imidazolylpropylamino pyrazolo-[1,5-a]-pyrimidine ;
et leurs sels d'addition avec un acide ou avec une base.

3. Composition selon la revendication 1 ou 2, **caractérisée par le fait que** les polyaminopyrimidines monocycliques sont choisies parmi la 2,4,5,6-tétra-aminopyrimidine, la 4-hydroxy 2,5,6-triaminopyrimidine, la 6-hydroxy 2,4,5-triaminopyrimidine, la 2-hydroxy 4,5,6-triaminopyrimidine, la 2,4-dihydroxy 5,6-diaminopyrimidine, la 2,6-dihydroxy 4,5-diaminopyrimidine, la 4,6-dihydroxy 2,5-diaminopyrimidine, la 2,5,6-triaminopyrimidine, la 2-diméthylamino 4,5,6-triaminopyrimidine, et leurs sels d'addition avec un acide.

4. Composition selon l'une quelconque des revendications 1 à 3, **caractérisée par le fait que** le ou les coupleurs sont choisis parmi les métaphénylènediamines, les méta-aminophénols, les métadiphénols et les coupleurs hétérocycliques.

5. Composition selon la revendication 4, **caractérisée par le fait que** le ou les coupleurs sont choisis parmi le 5-amino 2-méthyl phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 6-hydroxybenzomorpholine, la 2,6-dihydroxy 4-méthyl pyridine, le 1-H 3-méthyl pyrazole 5-one, le 1-phényl 3-méthyl pyrazole 5-one, et leurs sels d'addition avec un acide.

6. Composition selon l'une quelconque des revendications précédentes, **caractérisée par le fait que** les sels d'addition avec un acide sont choisis parmi les chlorhydrates, les bromhydrates, les sulfates, les citrates, les succinates, les tartrates, les lactates et les acétates et que les sels d'addition avec une base sont choisis parmi ceux obtenus avec la soude, la potasse, l'ammoniaque ou les amines.

7. Composition selon l'une quelconque des revendications précédentes, **caractérisée par le fait que** la ou les pyrazolo-[1,5-a]-pyrimidines de formule (I) et/ou le ou leurs sels d'addition avec un acide ou avec une base représentent de 0,0005 à 12% en poids du poids total de la composition tinctoriale.

8. Composition la revendication 7, **caractérisée par le fait que** la ou les pyrazolo-[1,5-a]-pyrimidines de formule (I) et/ou le ou leurs sels d'addition avec un acide ou avec une base représentent de 0,005 à 6% en poids du poids total de la composition tinctoriale.

9. Composition selon l'une quelconque des revendications précédentes, **caractérisée par le fait que** la ou les polyaminopyrimidines monocycliques et/ou le ou leurs sels d'addition avec un acide représentent de 0,0005 à 12 % en poids du poids total de la composition tinctoriale.

10. Composition selon la revendication 9, **caractérisée par le fait que** la ou les polyaminopyrimidines monocycliques et/ou le ou leurs sels d'addition avec un acide représentent de 0,005 à 6 % en poids du poids total de la composition tinctoriale.

11. Composition selon l'une quelconque des revendications précédentes, **caractérisée par le fait que** le ou les coupleurs représentent de 0,0001 % à 10% en poids du poids total de la composition tinctoriale.

12. Composition selon la revendication 11, **caractérisée par le fait que** le ou le coupleurs représentent de 0,005 à 5 % en poids du poids total de la composition tinctoriale.

13. Procédé de teinture d'oxydation des fibres kératiniques et en particulier des fibres kératiniques humaines telles que les cheveux **caractérisé par le fait qu'**on applique sur lesdites fibres au moins une composition tinctoriale telle que définie à l'une quelconque des revendications 1 à 12, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

14. Procédé selon la revendication 13, **caractérisé par le fait que** l'agent oxydant est choisi parmi le peroxyde d'hydrogène, le peroxyde d'urée, les bromates de métaux alcalins, les persels, les peracides, et les enzymes.

15. Dispositif à plusieurs compartiments, ou "kit" de teinture à plusieurs compartiments, dont un premier compartiment renferme une composition tinctoriale telle que définie à l'une quelconque des revendications 1 à 12 et un second compartiment renferme une composition oxydante contenant un agent oxydant.

## Patentansprüche

1. Zusammensetzung für die oxidative Färbung von Keratinfasern und insbesondere von menschlichen Keratinfasern, wie der Haare, **dadurch gekennzeichnet, dass** sie in einem für die Färbung geeigneten Medium enthält:
- mindestens eine Oxidationsbase, die unter den Pyrazolo[1, 5-a]pyrimidinen der folgenden Formel (I) und deren Additionssalzen mit einer Säure oder mit einer Base 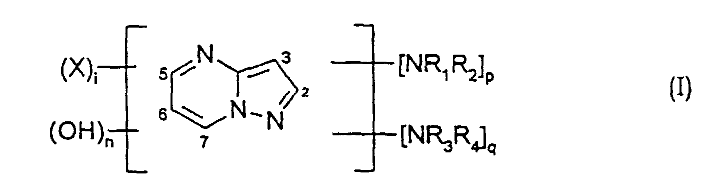 ausgewählt ist, in der bedeuten:
- R₁, R₂, R₃ und R₄, gleich oder verschieden, Wasserstoff, C₁₋₄-Alkyl, Aryl, C₁₋₄-Hydroxyalkyl, C₂₋₄-Polyhydroxyalkyl, (C₁₋₄)-Alkoxy-C₁₋₄-alkyl, C₁₋₄-Aminoalkyl (wobei das Amin durch eine Acetyl-, Ureido-, Sulfonylgruppe geschützt sein kann), (C₁₋₄)-Alkylamino-C₁₋₄-alkyl, Di-[(C₁₋₄)-alkyl]-amino-C₁₋₄-alkyl (wobei die Dialkyle einen aliphatischen oder heterocyclischen Ring mit 5 oder 6 Ringgliedern bilden können), (C₁₋₄)-Hydroxyalkyl- oder Di[(C₁₋₄)-hydroxyalkyl]-amino-C₁₋₄-alkyl;
- die Reste X, gleich oder verschieden, Wasserstoff, C₁₋₄-Alkyl, Aryl, C₁₋₄-Hydroxyalkyl, C₂₋₄-Polyhydroxyalkyl, C₁₋₄-Aminoalkyl, (C₁₋₄)-Alkylamino-C₁₋₄-alkyl, Di-[(C₁₋₄)-alkyl]-amino-C₁₋₄-alkyl (wobei die Dialkyle einen aliphatischen oder heterocyclischen Ring mit 5 oder 6 Ringgliedern bilden können), (C₁₋₄)-Hydroxyalkyl- oder Di[(C₁₋₄)-hydroxyalkyl]-amino-C₁₋₄-alkyl, Amino, (C₁₋₄)-Alkylamino oder Di-[(C₁₋₄)alkyl]-amino, ein Halogenatom, eine Carbonsäuregruppe, eine Sulfonsäuregruppe;
- i die Zahl 0, 1, 2 oder 3;
- p die Zahl 0 oder 1;
- q die Zahl 0 oder 1;
- n die Zahl 0 oder 1;
mit der Maßgabe, dass
- (i) die Summe p + q verschieden von 0 ist;
- (ii) n 0 bedeutet und die Gruppen NR₁R₂ und NR₃R₄ die Positionen (2,3); (5,6); (6,7); (3,5) oder (3,7) einnehmen, wenn p + q gleich 2 ist;
- (iii) n gleich 1 ist und die Gruppe NR₁R₂ (oder NR₃R₄) und die Gruppe OH die Positionen (2,3); (5,6); (6,7); (3,5) oder (3,7) einnehmen, wenn p + q gleich 1 ist;
- mindestens ein monocyclisches Polyaminopyrimidin als zweite Oxidationsbase; und
- mindestens einen Kuppler.

2. Zusammensetzung nach Anspruch 1, **dadurch gekennzeichnet, dass** die Pyrazolo[1,5-a]pyrimidine der Formel (I) ausgewählt sind unter:
- Pyrazolo[1,5-a]pyrimidin-3,7-diamin;
- 2-Methylpyrazolo[1,5-a]pyrimidin-3,7-diamin;
- 2,5-Dimethylpyrazolo[1,5-a]pyrimidin-3,7-diamin;
- Pyrazolo[1,5-a]pyrimidin-3,5-diamin;
- 2,7-Dimethylpyrazolo[1,5-a]pyrimidin-3,5-diamin;
- 3-Aminopyrazolo[1,5-a]pyrimidin-7-ol;
- 3-Amino-5-methylpyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 3-Amino-7-β-hydroxyethylamino-5-methylpyrazolo[1,5-a]pyrimidin;
- 2-(7-Arninopyrazolo[1,5-a]pyrimidin-3-ylamino)-ethanol;
- 2-[(3-Aminopyrazolo[1,5-a]pyrimidin-7-yl)-(2-hydroxyethyl)-amino)-ethanol;
- 2-[(7-Aminopyrazolo[1,5-a]pyrimidin-3-yl)-(2-hydroxyethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 2,5, N-7, N-7-Tetramethylpyrazolo[1,5-a]pyrimidin-3,7-diamin;
- 3-Amino-5-methyl-7-imidazolylpropylaminopyrazolo[1,5-a]-pyrimidin;
und deren Additionssalzen mit einer Säure oder mit einer Base.

3. Zusammensetzung nach Anspruch 1 oder 2, **dadurch gekennzeichnet, dass** die monocyclischen Polyaminopyrimidine ausgewählt sind unter 2,4,5,6-Tetraaminopyrimidin, 4-Hydroxy-2,5,6-triaminopyrimidin, 6-Hydroxy-2,4,5-triaminopyrimidin, 2-Hydroxy-4,5,6-triaminopyrimidin, 2,4-Dihydroxy-5,6-diaminopyrimidin, 2,6-Dihydroxy-4,5-diaminopyrimidin, 4,6-Dihydroxy-2,5-diaminopyrimidin, 2,5,6-Triaminopyrimidin, 2-Dimethylamino-4,5,6-triaminopyrimidin und deren Additionssalzen mit einer Säure.

4. Zusammensetzung nach einem der Ansprüche 1 bis 3, **dadurch gekennzeichnet, dass** der oder die Kuppler unter den m-Phenylendiaminen, den m-Aminophenolen, den m-Diphenolen und den heterocyclischen Kupplern ausgewählt sind.

5. Zusammensetzung nach Anspruch 4, **dadurch gekennzeichnet, dass** der oder die Kuppler ausgewählt sind unter 5-Amino-2-methylphenol, -N-(β-Hydroxyethyl)-amino-2-methylphenol, 3-Aminophenol, 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxyethylamino)-1-methoxybenzol, 1,3-Diaminobenzol, 1,3-Bis(2,4-diaminophenoxy)-propan, Sesamol, α-Naphthol, 6-Hydroxyindol, 4-Hydroxyindol, 4-Hydroxy-N-methylindol, 6-Hydroxyindolin, 6-Hydroxybenzomorpholin, 2,6-Dihydroxy-4-methylpyridin, 1-H-3-Methylpyrazol-5-on, 1-Phenyl-3-methylpyrazol-5-on, und deren Additionssalzen mit einer Säure.

6. Zusammensetzung nach einem der vorhergehenden Ansprüche, **dadurch gekennzeichnet, dass** die Additionssalze mit einer Säure unter den Hydrochloriden, den Hydrobromiden, den Sulfaten, den Citraten, den Succinaten, den Tartraten, den Lactaten und den Acetaten ausgewählt sind und dass die Additionssalze mit einer Base unter den Salzen ausgewählt sind, die mit Natriumhydroxid, Kaliumhydroxid, Ammoniak oder Aminen erhalten werden.

7. Zusammensetzung nach einem der vorhergehenden Ansprüche, **dadurch gekennzeichnet, dass** das oder die Pyrazolo[1,5-a]pyrimidine der Formel (I) und/oder das oder ihre Additionssalze mit einer Säure oder mit einer Base 0,0005 bis 12 Gew.-% des Gesamtgewichts der Farbmittelzusammensetzung ausmachen.

8. Zusammensetzung nach Anspruch 7, **dadurch gekennzeichnet, dass** das oder die Pyrazolo[1,5-a]pyrimidine der Formel (I) und/oder das oder ihre Additionssalze mit einer Säure oder mit einer Base 0,005 bis 6 Gew.-% des Gesamtgewichts der Farbmittelzusammensetzung ausmachen.

9. Zusammensetzung nach einem der vorhergehenden Ansprüche, **dadurch gekennzeichnet, dass** das oder die monocyclischen Polyaminopyrimidine und/oder das oder ihre Additionssalze mit einer Säure 0,0005 bis 12 Gew.-% des Gesamtgewichts der Farbmittelzusammensetzung ausmachen.

10. Zusammensetzung nach Anspruch 9, **dadurch gekennzeichnet, dass** das oder die monocyclischen Polyaminopyrimidine und/oder das oder ihre Additionssalze mit einer Säure 0,005 bis 6 Gew.-% des Gesamtgewichts der Farbmittelzusammensetzung ausmachen.

11. Zusammensetzung nach einem der vorhergehenden Ansprüche, **dadurch gekennzeichnet, dass** der oder die Kuppler 0,0001 bis 10 Gew.-% des Gesamtgewichts der Farbmittelzusammensetzung ausmachen.

12. Zusammensetzung nach Anspruch 11, **dadurch gekennzeichnet, dass** der oder die Kuppler 0,005 bis 5 Gew.-% des Gesamtgewichts der Farbmittelzusammensetzung ausmachen.

13. Verfahren zur oxidativen Färbung von Keratinfasern und insbesondere von menschlichen Keratinfasern, wie der Haare, **dadurch gekennzeichnet, dass** auf diese Fasern mindestens eine wie in einem der Ansprüche 1 bis 12 definierte Farbmittelzusammensetzung aufgetragen wird und dass die Farbe bei saurem, neutralem oder alkalischem pH-Wert mit Hilfe eines Oxidationsmittels entwickelt wird, das im Augenblick der Verwendung zu der Farbmittelzusammensetzung geben wird oder das in einer oxidierenden Zusammensetzung enthalten ist, die gleichzeitig oder sequentiell aufgetragen wird.

14. Verfahren nach Anspruch 13, **dadurch gekennzeichnet, dass** das Oxidationsmittel unter Wasserstoffperoxid, Harnstoffperoxid, den Alkalimetallbromaten, den Persalzen, den Persäuren und den Enzymen ausgewählt wird.

15. Vorrichtung mit mehreren Abteilungen oder "Kit" zum Färben mit mehreren Abteilungen, von denen eine erste Abteilung eine wie in einem der Ansprüche 1 bis 12 definierte Farbmittelzusammensetzung enthält und eine zweite Abteilung eine oxidierende Zusammensetzung enthält, die ein Oxidationsmittel enthält.

## Claims

1. Composition for the oxidation dyeing of keratin fibres, and in particular human keratin fibres such as the hair, **characterized in that** it contains, in a medium which is suitable for dyeing:
- at least one oxidation base chosen from pyrazolo[1,5-a]pyrimidines of formula (I) below, and the addition salts thereof with an acid or with a base: 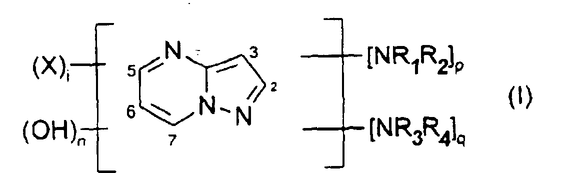 in which:
- R₁, R₂, R₃ and R₄, which may be identical or different, denote a hydrogen atom; a C₁-C₄ alkyl radical; an aryl radical; a C₁-C₄ hydroxyalkyl radical; a C₂-C₄ polyhydroxyalkyl radical; a (C₁-C₄)alkoxy (C₁-C₄)alkyl radical; a C₁-C₄ aminoalkyl radical (it being possible for the amine to be protected with an acetyl, a ureido or a sulphonyl); a (C₁-C₄) alkylamino (C₁-C₄)alkyl radical; a di [(C₁-C₄) alkyl] amino (C₁-C₄ )alkyl radical (it being possible for the dialkyls to form a 5- or 6-membered aliphatic or heterocyclic ring); a hydroxy(C₁-C₄)alkyl- or di[hydroxy (C₁-C₄)alkyl]amino(C₁-C₄)alkyl radical;
- the radicals X, which may be identical or different, denote a hydrogen atom, a C₁-C₄ alkyl radical, an aryl radical, a C₁-C₄ hydroxyalkyl radical, a C₂-C₄ polyhydroxyalkyl radical, a C₁-C₄ aminoalkyl radical, a (C₁-C₄)alkylamino(C₁-C₄)alkyl radical, a di[(C₁-C₄)alkyl]amino(C₁-C₄)alkyl radical (it being possible for the dialkyls to form a 5- or 6-membered aliphatic or heterocyclic ring), a hydroxy(C₁-C₄)alkyl- or di[hydroxy(C₁-C₄)alkyl]amino(C₁-C₄)alkyl radical, an amino radical, a (C₁-C₄)alkyl- or di[(C₁-C₄)-alkyllamino radical; a halogen atom, a carboxylic acid group or a sulphonic acid group;
- i is 0, 1, 2 or 3;
- p is 0 or 1;
- q is 0 or 1;
- n is 0 or 1;
with the proviso that:
- (i) the sum p + q is other than 0;
- (ii) when p + q is equal to 2, then n is 0 and the groups NR₁R₂ and NR₃R₄ occupy positions (2,3); (5,6); (6, 7); (3,5) or (3,7);
- (iii) when p + q is equal to 1, then n is 1 and the group NR₁R₂ (or NR₃R₄) and the OH group occupy positions (2,3); (5,6): (6, 7); (3,5) or (3, 7);
- at least one monocyclic polyaminopyrimidine as second oxidation base;
- and at least one coupler.

2. Composition according to Claim 1, **characterized in that** the pyrazolo[1,5-a]pyridimines of formula (I) are chosen from:
- pyrazolo[1,5-a]pyrimidine-3,7-diamine;
- 2-methylpyrazolo[1,5-a]pyrimidine-3,7-diamine;
- 2,5-dimethylpyrazolo[1,5-a]pyrimidine-3,7-diamine;
- pyrazolo[1,5-a]pyrimidine-3,5-diamine;
- 2,7-dimethylpyrazolo[1,5-a]pyrimidine-3,5-diamine;
- 3-aminopyrazolo[1,5-a]pyrimidin-7-ol;
- 3-amino-5-methylpyrazolo[2,5-a]pyrimidin-7-ol;
- 3-aminopyrazolo[1,5-a]pyrimidin-5-ol;
- 2-(3-aminopyrazolo[1,5-a]pyrimidin-7-ylamino)-ethanol;
- 3-amino-7-β-hydroxyethylamino-5-methylpyrazolo[1,5-a]pyrimidine;
- 2-(7-aminopyrazolo[1,5-a]pyrimidin-3-ylamino)-ethanol;
- 2-[(3-aminopyrazolo[1,5-a]pyrimidin-7-yl)-(2-hydroxyethyl)amino]ethanol;
- 2-[(7-aminopyrazolo[1,5-a]pyrimidin-3-yl)-(2-hydroxyethyl)amino]ethanol;
- 5, 6-dimethylpyrazolo[1,5-a]pyrimidine-3,7-diamine;
- 2,6-dimethylpyrazolo[1,5-a]pyrimidine-3,7-diamine;
- 2,5,N-7,N-7-tetramethylpyrazolo[1,5-a]pyrimidine-3,7-diamine;
- 3-amino-5-methyl-7-imidazolylpropylaminopyrazolo[1,5-a]pyrimidine;
and the addition salts thereof with an acid or with a base.

3. Composition according to Claim 1 or 2, **characterized in that** the monocyclic polyaminopyrimidines are chosen from 2,4,5,6-tetraaminopyrimidine, 4-hydroxy-2,5,6-triaminopyrimidine, 6-hydroxy-2,4,5-triaminopyrimidine, 2-hydroxy-4,5,6-triaminopyrimidine, 2,4-dihydroxy-5,6-diaminopyrimidine, 2,6-dihydroxy-4,5-diaminopyrimidine, 4,6-dihydroxy-2,5-diaminopyrimidine, 2,5,6-triaminopyrimidine and 2-dimethylamino-4,5,6-triaminopyrimidine, and the addition salts thereof with an acid.

4. Composition according to any one of Claims 1 to 3, **characterized in that** the coupler(s) is (are) chosen from meta-phenylenediamines, meta-aminophenols, meta-diphenols and heterocyclic couplers.

5. Composition according to Claim 4, **characterized in that** the coupler(s) is (are) chosen from 5-amino-2-methylphenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)-benzene, 2-amino-4-(β-hydroxyethylamino)-1-methoxybenzene, 1,3-diaminobenzene, 1,3-bis(2,4-diaminophenoxy)propane, sesamol, α-naphthol, 6-hydroxyindole, 4-hydroxyindole, 4-hydroxy-N-methylindole, 6-hydroxyindoline, 6-hydroxybenzomorpholine, 2,6-dihydroxy-4-methylpyridine, 1-H-3-methylpyrazol-5-one and 2-phenyl-3-methylpyrazol-5-one, and the addition salts thereof with an acid.

6. Composition according to any one of the preceding claims, **characterized in that** the addition salts with an acid are chosen from the hydrochlorides, hydrobromides, sulphates, citrates, succinates, tartrates, lactates and acetates, and **in that** the addition salts with a base are chosen from those obtained with sodium hydroxide, potassium hydroxide, aqueous ammonia or amines.

7. Composition according to any one of the preceding claims, **characterized in that** the pyrazolo[1,5-a]pyrimidine(s) of formula (I) and/or the addition salt(s) thereof with an acid or with a base represent from 0.0005 to 12% by weight relative to the total weight of the dye composition.

8. Composition according to Claim 7, **characterized in that** the pyrazolo[1,5-a]pyrimidine(s) of formula (I) and/or the addition salt(s) thereof with an acid or with a base represent from 0.005 to 6% by weight relative to the total weight of the dye composition.

9. Composition according to any one of the preceding claims, **characterized in that** the monocyclic polyaminopyrimidine(s) and/or the addition salt(s) thereof with an acid represent from 0.0005 to 12% by weight relative to the total weight of the dye composition.

10. Composition according to Claim 9, **characterized in that** the monocyclic polyaminopyrimidine(s) and/or the addition salt(s) thereof with an acid represent from 0.005 to 6% by weight relative to the total weight of the dye composition.

11. Composition according to any one of the preceding claims, **characterized in that** the coupler(s) represent(s) from 0.0001 to 10% by weight relative to the total weight of the dye composition.

12. Composition according to Claim 11, **characterized in that** the coupler(s) represent(s) from 0.005 to 5% by weight relative to the total weight of the dye composition.

13. Process for oxidation dyeing keratin fibres, and in particular human keratin fibres such as the hair, **characterized in that** at least one dye composition as defined in any one of Claims 1 to 12 is applied to the said fibres, and **in that** the colour is developed at acidic, neutral or alkaline pH with the aid of an oxidizing agent which is added to the dye composition just at the time of use, or which is present in an oxidizing composition that is applied simultaneously or sequentially.

14. Process according to Claim 13, **characterized in that** the oxidizing agent is chosen from hydrogen peroxide, urea peroxide, alkali metal bromates, persalts, peracids and enzymes.

15. Multi-compartment dyeing device or multicompartment dyeing kit, a first compartment of which contains a dye composition as defined in any one of Claims 1 to 12 and a second compartment of which contains an oxidizing composition containing an oxidizing agent.
